# EUROPEAN PATENT SPECIFICATION

(11) **EP 4 297 812 B1**
(45) Date of publication and mention of the grant of the patent: **04.11.2026**
(21) Application number: 22711372.7
(22) Date of filing: 25.02.2022
(51) Int. Cl.: A61L 29/06, A61L 29/14, A61L 29/16, C08G 18/08, C08G 18/32, C08G 18/38, C08G 18/48, C08G 18/50, C08G 18/61, C08G 18/66, C08L 75/08, C08G 18/76

(54) **POLYURETHANE BASED MEDICAL ARTICLES**
MEDIZINISCHE ARTIKEL AUF POLYURETHANBASIS
ARTICLES MÉDICAUX À BASE DE POLYURÉTHANE

(30) Priority: 25.02.2021 US 202163153805 P
(43) Date of publication of application: 03.01.2024
(73) Proprietor: Becton, Dickinson and Company, Franklin Lakes, NJ 07417-1880 (US)
(72) Inventor: BAI, He, Sandy, Utah 84093 (US); WEIMER, Marc W., South Jordan, Utah 84095 (US); FREASIER, James, Salt Lake City, Utah 84109 (US)
(74) Representative: dompatent
(86) International application number: PCT/US2022/017870
(87) International publication number: WO 2022/182969

(56) References cited:
- EP-A2- 0 184 465
- US-A1- 2018 146 665
- US-A1- 2019 106 525
- US-A1- 2020 095 515

## Description

### TECHNICAL FIELD

The present disclosure relates to a polyurethane-based resin including a backbone of a diisocyanate, a polyglycol, and a diol chain extender, which also includes addition of at least one ionically-charged modifier into the backbone, or both incorporated into a backbone and as a side chain of the polyurethane-based resin formed by the diisocyanate, the polyglycol, and the diol chain extender. The ionically-charged modifier is cationic, comprising one or more quaternary ammonium functional groups. Medical articles made therefrom either have inherent antimicrobial and/or anti-fouling characteristics or can easily bond anionic active agents to provide desirable material properties, including antimicrobial and anti-fouling.

### BACKGROUND

Infusion therapy medical devices, such as syringe cannulas and catheters used for sampling or medicament administration, typically have components that are in direct contact of bodily fluid that can cause infection. For example, catheter-related bloodstream infections may be caused by colonization of microorganisms, which can occur in patients whose treatment includes intravascular catheters and I.V. access devices. These infections can lead to illness and excess medical costs. Impregnating and/or coating catheters with various antimicrobial agents (e.g., chlorhexidine, silver or other antibiotics) is a common approach that has been implemented to prevent these infections.

Some blood contact devices have the potential to generate thrombus. When blood contacts a foreign material, a complex series of events occur. These involve protein deposition, cellular adhesion and aggregation, and activation of blood coagulation schemes. Thrombogenicity has conventionally been counteracted by the use of anticoagulants such as heparin. Attachment of heparin to otherwise thrombogenic polymeric surfaces may be achieved with various surface coating techniques.

Impregnating catheters directly with antimicrobial/antithrombogenic agents does not create chemical bonding between active agents and polymer substrates, thus devices would lose antifouling efficacy in a short time and it would also create regulatory concerns, e.g., heparin-induced thrombocytopenia (HIT). Surface coating techniques are to heparinize the polymer substrate or bond an antibiotic to the polymer substrate by chemical bonding to achieve non-leaching or controlled release of active agents. However, these coating techniques would require priming of polymer substrates (e.g., chemical or plasma treatments), followed by multiple steps of surface coating, which would complicate the medical device manufacturing process and significantly increase manufacturing costs.

Thus, there is a need for polymeric resins, in particular polyurethane resins, that either has inherent antimicrobial and/or anti-fouling characteristics or can easily bond antimicrobial/antithrombogenic agents to achieve antimicrobial and/or anti-fouling characteristics.

US 2018/146665 describes an antimicrobial polymer including a quaternary ammonium moiety within a backbone of said polymer to provide it with an antimicrobial character. A monomer having a quaternary ammonium group can be reacted with one or more diisocyanates, polyols and chain extenders to form said polymer in a two-step and solvent process.

### SUMMARY

One or more embodiments are directed to a medical article formed from a polyurethane-based resin, which is a reaction product of ingredients comprising: a diisocyanate; a diol chain extender; a polyglycol; and a cationic modifier comprising one or more quaternary ammonium functional groups, wherein the cationic modifier is incorporated into a backbone, or both incorporated into a backbone and as a side chain of the polyurethane-based resin formed by the diisocyanate, the polyglycol, and the diol chain extender, wherein the polyurethane-based resin is a random copolymer based on the ingredients, and wherein the polyurethane-based resin has a hard segment content in a range of from 25% to 75% by weight and a soft segment content of the resin is in a range of from 75% to 25% by weight.

An additional embodiment is directed to a medical article formed from a polyurethane-based resin, which is a reaction product of ingredients consisting essentially of: 4,4'-diphenylmethane diisocyanate (MDI) as the diisocyanate; 1,4-butanediol as the diol chain extender; a polytetramethylene ether glycol as the polyglycol; and bis(2-hydroxyethyl)dimethylammonium chloride (BHDAC) as the cationic modifier.

Further embodiments are directed to a medical article comprising a polyurethane-based resin that is a random copolymer comprising chain segments of (A), (B), and (C) as follows: 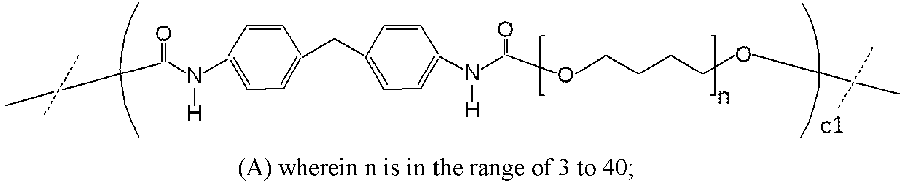 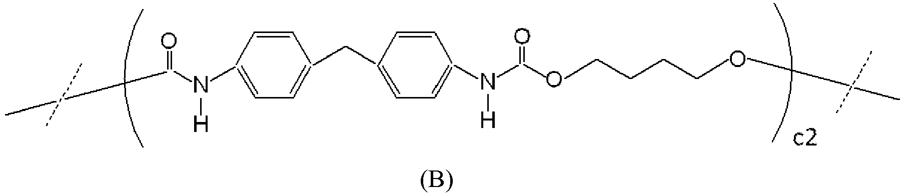 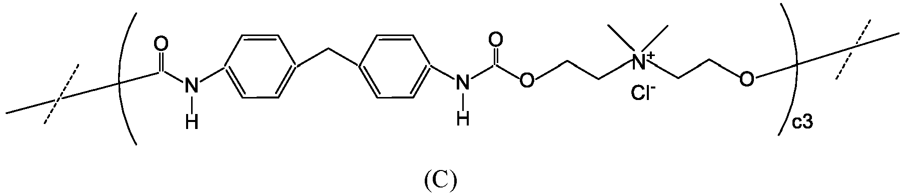 wherein a hard segment content is in the range of from 25% to 75% by weight and a soft segment content of the resin is in the range of from 75% to 25% by weight; the polyurethane-based resin has an overall ion exchange capacity of 0.01 to 1 mmol/g.

Additional embodiments described herein, but not forming part of the claimed invention, are directed to methods of infusion therapy comprising: infusing a material from a medical article according to any embodiment herein into a patient.

### BRIEF DESCRIPTION OF THE DRAWINGS

FIG. 1 is a thermogravimetric analysis (TGA) curve, weight (%) versus temperature (°C) for an embodiment;
FIG. 2 is a thermogravimetric analysis (TGA) curve, weight (%) versus temperature (°C) for an embodiment;
FIG. 3 is a thermogravimetric analysis (TGA) curve, weight (%) versus temperature (°C) for a reference embodiment; and
FIG. 4 is a plan view of an exemplary medical device.

### DETAILED DESCRIPTION

The scope of this invention is defined by the claims. Embodiments in the description relating to methods of treatment are not covered by the claims. Any "embodiment" or "example" which is disclosed in the description but is not covered by the claims should be considered as presented for illustrative purpose only. Before describing several exemplary embodiments of the invention, it is to be understood that the invention is not limited to the details of construction or process steps set forth in the following description. The invention is capable of other embodiments and of being practiced or being carried out in various ways.

The following terms shall have, for the purposes of this application, the respective meanings set forth below.

Polyglycols include but are not limited to: polyalkylene glycol, polyester glycol, and polycarbonate glycol. A non-limiting specific example of polyalkylene glycol is polyether glycol. A polyether glycol is a moderate molecular weight oligomer derived from an alkylene oxide, containing both ether linkages and glycol termination.

A chain extender is a short chain (low molecular weight) branched or unbranched diol, diamine or amino alcohol of up to 10 carbon atoms or mixtures thereof. Such hydroxyl- and/or amine-terminated compounds are used during polymerization to impart desired properties to a polymer.

An ionically-charged modifier is a compound exhibiting a charge that enhances a basic polyurethane structure of a diisocyanate; a diol chain extender; and a polyglycol. The ionically-charged modifier according to the claimed invention comprises a cationic modifier, comprising one or more quaternary ammonium functional groups that make the polyurethane cationic in nature to render the resulting medical article with desirable properties. The desired properties include passive reduction of bacterial biofilm colony formation due to inhibition of microbial growth by cationic quaternary ammonium and antifouling property due to ionic repulsion of blood components. The cationic modifier is incorporated into a backbone, or both into a backbone and as a side chain of the polyurethane-based resin.

Antimicrobial agents that can be used for bonding with cationic functional moieties of the polyurethane include any anionic antibiotics, e.g., cloxacillin salt, cefoxitin salt, cefazolin salt, penicillin salt, or derivatives thereof. Similarly, anionic antithrombogenic agents, e.g., heparin salt, can be ionically bonded with cationic functional moieties of the polyurethane to provide medical article desirable antithrombogenic properties. In addition, the skilled artisan will recognize that other anionic biocides and anticoagulants of either small molecules or macromolecules can also be used for bonding with cationic functional groups of the polyurethane.

A low-surface energy modifying oligomer (moderate molecular weight), as described in WO 2020/068617 A1 and WO 2020/068619 A1, which is optional in embodiments herein, is a compound that enhances a basic polyurethane structure of a diisocyanate; a diol chain extender; a polyglycol; and a cationic modifier. Modifying oligomers, which are different from polyglycols and a cationic modifier, contain functional moieties (e.g., fluoroether and/or silicone) that migrate onto the polyurethane surface to render the resulting medical article with additional desirable surface properties including self-lubricating and antifouling property. Modifying oligomers may have at least one, preferably two, or more than two, alcohol moieties (C-OH). The alcohol moieties may be located along a backbone of the oligomer. The alcohol moieties may be located at an end of the oligomer. In a detailed embodiment, the oligomer terminates with an alcohol moiety.

Isocyanate index is defined as the molar ratio of the total isocyanate groups in the diisocyanate to the total hydroxyl and/or amino groups presented in polyols and extenders. In general, the polyurethane becomes harder with an increasing isocyanate index. There is, however, a point beyond which the hardness does not increase and the other physical properties begin to deteriorate.

As used herein, the term "consists essentially of" means that the material does not contain any other components in amounts that may alter the properties of the polyurethane material.

Principles and embodiments of the present disclosure relate generally to thermoplastic polyurethane (TPU) materials having improved properties, and methods of preparing and using them. Provided are medical articles, for example, catheter tubing, that either have inherent antimicrobial and/or anti-fouling characteristics or can easily bond anionic active agents to provide desirable material properties, including antimicrobial and anti-fouling. Included with traditional polyurethane monomers is an ionically-charged modifier. Herein, the ionically-charged modifier is cationic, whose functional moieties (i.e. quaternary ammonium) can be introduced into soft segments of the TPU materials using polyglycols and/or optional low-surface energy modifying oligomers with cationic functionalities or hard segments of TPU materials using diol chain extenders and/or diisocyanates with cationic functionalities.

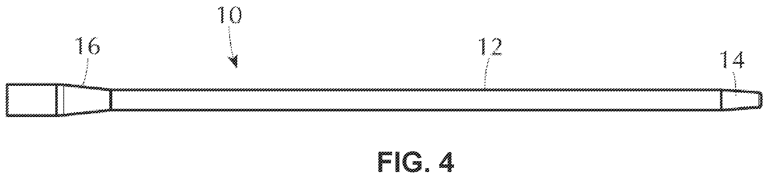

In FIG. 4, an exemplary medical article in the form of a catheter is illustrated. Tubing made from polyurethane resins as disclosed herein forms the catheter, which is shaped as needed to receive other components for forming vascular access devices. Catheter 10 comprises a primary conduit 12, which is tubing in its as-extruded form. At a distal end, a tip 14 is formed by a tipping process. At a proximal end, a flange 16 is formed as needed for receipt of other components including but not limited to catheter adapters. Exemplary vascular access devices may include a needle further to the catheter for access to blood vessels.

The articles comprise a polyurethane-based resin that is a reaction product of the following ingredients: a diisocyanate; a diol chain extender; a polyglycol; and a cationic modifier incorporated into a backbone of the polyurethane-based resin, or both into a backbone and as a side chain of the polyurethane-based resin. Incorporation into backbone means that cationic functionalities (i.e. quaternary ammonium) are directly linked to the polyurethane backbone chain; incorporation as a side chain means that there is at least one carbon chain spacer between cationic functionalities and the polyurethane backbone chain. The polyurethane-based resin is a random copolymer based on the ingredients and comprises a hard segment content in a range of from 25% to 75% by weight and a soft segment content of the resin in a range of from 75% to 25% by weight. In one or more embodiments, the polyurethane-based resin has an overall ion exchange capacity in a range of from 0.01 to 1 mmol/g.

In one or more embodiments, the cationic modifier is incorporated into the polyurethane-based resin in an amount of greater than or equal to: 0.01 wt. %, 0.1 wt. %, 0.5 wt. %, 1 wt. %, 1.5 wt. %, 2 wt. %, 3 wt. %, 4 wt. % and 4.5 wt.% of the overall composition of the polyurethane-based resin. In one or more embodiments, the cationic modifier is incorporated into the polyurethane-based resin in an amount of less than or equal to: 10 wt. %, 9.5 wt. %, 9.0 wt. %, 8.5 wt. %, 8.0 wt. %, 7.5 wt. %, 7.0 wt. %, 6.5 wt. % or 6.0 wt. % of the overall composition of the polyurethane-based resin. In one or more embodiments, the cationic modifier is incorporated into the polyurethane-based resin in an amount ranging from greater than or equal to 0.01 to less than or equal to 10 wt. %, and all values and subranges therebetween, including greater than or equal to 0.5 to less than or equal to 7.5 wt. %, greater than or equal to 1.0 to less than or equal to 6.0 wt. %, and all values and subranges there between; including: greater than or equal to: 0.01 wt. %, 0.1 wt.%, 0.5 wt. %, 1 wt. %, 1.5 wt.%, 2 wt. %, 3 wt. %, 4 wt. % and 4.5 wt.% to less than or equal to: 10 wt. %, 9.5 wt.%, 9.0 wt. %, 8.5 wt. %, 8.0 wt. %, 7.5 wt. %, 7.0 wt. %, 6.5 wt. %, 6.0 wt. % of the overall composition of the polyurethane-based resin.

According to the claimed invention, the cationic modifier comprises one or more quaternary ammonium functional moieties. A non-limiting example of the cationic modifier with quaternary ammonium functional moiety is bis(2-hydroxyethyl)dimethylammonium chloride (BHDAC).

According to the claimed invention, the cationic modifier is incorporated into the backbone. Non-limiting examples of the cationic modifier incorporated into the backbone include bis(2-hydroxyethyl)dimethylammonium chloride (BHDAC).

In one or more embodiments, the cationic modifier is incorporated both as a side chain and into the backbone, as discussed herein.

In an embodiment, the polyurethane-based resin is a reaction product of: a diisocyanate; a diol chain extender; a polyglycol; and a bis(2-hydroxyethyl)dimethylammonium chloride (BHDAC). In an embodiment, the polyurethane-based resin is a reaction product of: a diisocyanate; a diol chain extender; a polyglycol; and combination of two or more cationic modifiers.

In a detailed embodiment, the polyurethane-based resin is a reaction product of ingredients consisting essentially of: 4,4'-diphenylmethane diisocyanate (MDI) as the diisocyanate; 1,4-butanediol as the diol chain extender; polytetramethylene ether glycol(s) as the polyglycols; and bis(2-hydroxyethyl)dimethylammonium chloride (BHDAC) as the cationic modifier.

In a detailed embodiment, the polyurethane-based resin is a reaction product of: a diisocyanate; a diol chain extender; a polyglycol; a cationic modifier incorporated into a backbone, or both into a backbone and as a side chain of the polyurethane-based resin; and a low-surface energy modifying oligomer (as described in WO 2020/068617 A1 and WO 2020/068619 A1) incorporated into a backbone, as a side chain, or both of the polyurethane-based resin.

The polyurethane-based resins herein are synthesized by a conventional one-step copolymerization process. Catalyst or solvent may be required. The synthesis can also be achieved by a variety of other synthesis techniques with or without catalyst/solvent understood by those skilled in the art. Through structural and compositional design, the resulting cationic polyurethane resins can potentially possess inherent antimicrobial and/or anti-fouling surface properties for medical device applications, due to inhibition of microbial growth by cationic quaternary ammonium and ionic repulsion of blood components.

Antimicrobial agents that can be used for bonding with cationic functional moieties of the polyurethane include any anionic antibiotics. Non-limiting examples of the anionic antibiotics include cloxacillin salt, cefoxitin salt, cefazolin salt, penicillin salt, or derivatives thereof. Non-limiting examples of the anionic antithrombogenic agents include heparin salt, or derivatives thereof. In addition, the skilled artisan will recognize that other anionic biocides and anticoagulants of either small molecules or macromolecules can also be used for bonding with cationic functional groups of the polyurethane. Ionic bonding of active agents can be achieved by solution imbibing technique or bulk mixing (e.g., thermal compounding or solvent mixing) technique. As a result, anionic antimicrobial and/or anionic antithrombogenic agents would be ionically bonded not only on cationic TPU surface but also in the bulk cationic TPU to render the resulting medical device desirable properties, including antimicrobial and anti-fouling.

### POLYURETHANES

Polyurethane materials disclosed herein have enhanced surface properties, which may be tailored to fit different practical needs. Medical devices formed of these polyurethane materials are used to create a fluid channel from a medication reservoir to a patient in need thereof, where the fluid channel may be inserted into and in fluid communication with vascular vessels, or subcutaneous tissue, where the invasive medical device comprises any of the polyurethane materials as described herein.

Thermoplastic polyurethanes (TPUs) suitable for medical devices are typically synthesized from three basic components, a diisocyanate, a polyglycol, and a chain extender, usually a low molecular weight diol, diamine, amino alcohol or water. If the chain extender is a diol, the polyurethane consists entirely of urethane linkages. If the extender is water, amino alcohol or diamine, both urethane and urea linkages are present, which results in a polyurethaneurea (PUU). Inclusion of an amine-terminated polyether to the polyurethane synthesis also results in a polyurethaneurea. Device applications for thermoplastic polyurethanes include central venous catheters (CVCs), peripherally inserted central catheter (PICCs), and peripheral intravenous catheters (PIVCs).

Polyurethane and polyurea chemistries are based on the reactions of isocyanates with other hydrogen-containing compounds, where isocyanates are compounds having one or more isocyanate group (-N=C=O). Isocyanate compounds can be reacted with water (H₂O), alcohols (R-OH), amines (Rₓ-NH₍₃₋ₓ₎), ureas (R-NH-CONH₂), and amides (R-CONH₂). Certain polyurethanes may be thermoplastic elastomers (TPE), whereas other compositions may be highly cross-linked.

Thermoplastic polyurethanes comprise two-phases or microdomains conventionally termed hard segments and soft segments, and as a result are often referred to as segmented polyurethanes. The hard segments, which are generally of high crystallinity, form by localization of the portions of the polymer molecules which include the diisocyanate and chain extender(s). The soft segments, which are generally either non-crystalline or of low crystallinity, form from the polyglycol or the optional amine-terminated polyether. The hard segment content is determined by the weight percent of diisocyanate and chain extender in the polyurethane composition, and the soft segment content is the weight percent of polyglycol or polydiamine. The thermoplastic polyurethanes may be partly crystalline and/or partly elastomeric depending on the ratio of hard to soft segments. One of the factors which determine the properties of the polymer is the ratio of hard and soft segments. In general, the hard segment contributes to hardness, tensile strength, impact resistance, stiffness and modulus while the soft segment contributes to water absorption, elongation, elasticity and softness.

Polyurethane materials may be used as raw materials for catheter tubing via compounding, extrusion/coextrusion or molding.

The polyurethanes are produced by the reaction of: a diisocyanate, a diol chain extender, at least one polyglycol, an ionically-charged modifier comprising one or more quaternary ammonium functional groups, and optionally, a low-surface energy modifying oligomer. The polyurethane has a hard segment content between 25% and 75% by weight, where a hard segment is the portion(s) of the polymer molecules which include the diisocyanate and the extender components, which are generally highly crystalline due to dipole-dipole interactions and/or hydrogen bonding. In contrast, the soft segments formed from the polyglycol portions and optionally the low-surface energy modifying oligomers between the diisocyanate of the polymer chains and generally are either amorphous or only partially crystalline due to the characteristics of the polyglycol(s) and modifying oligomer(s). According to the claimed invention, the hard segment content is in the range of from 25% to 75% and the soft segment content is in the range of from 75% to 25%. Herein, the ionically-charged modifier is cationic, whose cationic functional moieties can be introduced into soft segments of the TPU materials using polyglycols and/or optional low-surface energy modifying oligomers with cationic functionalities or hard segments of TPU materials using diol chain extenders and/or diisocyanates with cationic functionalities. The cationic functional moieties include quaternary ammonium. In an embodiment, ionically-charged modifier is introduced into hard segment of the TPU material using diol chain extender with cationic functionalities, i.e., bis(2-hydroxyethyl)dimethylammonium chloride (BHDAC).

Polymerization of the polyurethane may be a one-step copolymerization process. The process may require a catalyst, solvent, other additives, or a combination thereof. The synthesis can also be achieved by a variety of other synthesis techniques with or without catalyst/solvent understood by those skilled in the art.

The diisocyanate may be selected from the group consisting of: an aliphatic diisocyanate, alicyclic diisocyanate and an aromatic diisocyanate. In various embodiments, the diisocyanate may be selected from the group consisting of: 4,4'-diphenylmethane diisocyanate (MDI), toluene diisocyanate (TDI), isophorone diisocyanate (IPDI), methylene-bis(4-cyclohexylisocyanate) (HMDI), or combinations thereof.

The diol chain extender may be selected from the group consisting of: ethylene glycol, 1,3-propylene glycol, 1,4-butanediol, neopentyl glycol, and alicyclic glycols having up to 10 carbon atoms.

The polyglycol may be selected from the group consisting of: polyalkylene glycol, polyester glycol, polycarbonate glycol, and combinations thereof. In an embodiment, the polyglycol comprises the polyalkylene glycol. In an embodiment, the polyalkylene glycol comprises a polytetramethylene ether glycol.

The polytetramethylene ether glycol may be of any desired molecular weight. The desired molecular weight is the molecular weight in the range of from 200 Da to 4000 Da, or 250 Da to 2900 Da. The polytetramethylene ether glycol (PTMEG) may be PTMEG250, PTMEG650, PTMEG1000, PTMEG1400, PTMEG1800, PTMEG2000, and PTMEG2900. PTMEG has the formula: HO(CH₂CH₂CH₂CH₂-O-)ₙH, which may have an average value of n in the range of 3 to 40. A blend of two or more PTMEG250, PTMEG650, PTMEG1000, PTMEG1400, PTMEG1800, PTMEG2000, and PTMEG2900 may be used such. Reference to PTMEG250 means a polytetramethylene ether glycol having an average molecular weight in a range of 230 to 270 Da. Reference to PTMEG650 means a polytetramethylene ether glycol having an average molecular weight in a range of 625 to 675 Da. Reference to PTMEG1000 means a polytetramethylene ether glycol having an average molecular weight in a range of 950 to 1050 Da. Reference to PTMEG1400 means a polytetramethylene ether glycol having an average molecular weight in a range of 1350 to 1450 Da. Reference to PTMEG1800 means a polytetramethylene ether glycol having an average molecular weight in a range of 1700 to 1900 Da. Reference to PTMEG2000 means a polytetramethylene ether glycol having an average molecular weight in a range of 1900 to 2100 Da. Reference to PTMEG2900 means a polytetramethylene ether glycol having an average molecular weight in a range of 2825 to 2976 Da. In an embodiment, a preferred an average molecular weight of the combination is less than 1000 Da. In an embodiment, the polyol is a blend of two or more PTMEG having the formula: HO(CH₂CH₂CH₂CH₂-O-)ₙH, where n has an average value in the range of 3 to 40. In one or more embodiments, the polyols is a blend of two or more PTMEG having the formula: HO(CH₂CH₂CH₂CH₂-O-)ₙH, where n has an average value in the range of 3 to 40 and an average molecular weight of the combination being less than 1000 Da.

A further polyalkylene glycol may be polyethylene glycol (PEG) and/or polypropylene glycol (PPG). The PEG and/or PPG may comprise any desired molecular weight. The desired molecular weight is the average molecular weight in the range of from 200 Da to 8000 Da.

The polyurethane-based resin may further comprise a polyetheramine. Suitable polyetheramines include but are not limited to amine-terminated polyethers having repeating units of ethylene oxide, propylene oxide, tetramethylene oxide or combinations thereof and having an average molecular weight in the range of about 230 to 4000 Da. Preferred polyetheramines have propylene oxide repeating units. Jeffamine^{®} D4000 is a specific polyetheramine, a polyoxypropylene diamine, having an average molecular weight of about 4000 Da.

The ionically-charged modifier is cationic, containing cationic functional moieties (i.e. quaternary ammonium) that make the polyurethane cationic in nature. Resulting medical articles may advantageously have desirable surface properties including but not limited to antimicrobial and/or anti-fouling properties, due to inhibition of microbial growth by cationic quaternary ammonium and ionic repulsion of blood components.

Including an ionically-charged modifier such as a cationic modifier in the polyurethane resin such that a separate surface coating process to introduce antimicrobial/antithrombogenic agents may not be needed, can offer the following advantages: (i) simple cationic TPU copolymer composition with passive non-fouling surface, without leach-out concern of the active agents; (ii) no capital investment for coating process; (iii) much reduced manufacturing/conversion costs; (iv) less environment, health and safety (EHS) impact; (v) less regulatory concern, e.g., heparin-induced thrombocytopenia (HIT).

Antimicrobial agents that can be used for bonding with cationic functional moieties of the polyurethane include any anionic antibiotics. Non-limiting examples of anionic antibiotics include: cloxacillin salt, cefoxitin salt, cefazolin salt, penicillin salt and derivatives thereof. Non-limiting examples of the anionic antithrombogenic agents include heparin salt, or derivatives thereof. In addition, the skilled artisan will recognize that other anionic biocides and anticoagulants of either small molecules or macromolecules can also be used for bonding with cationic functional groups of the polyurethane.

Should an antimicrobial/antithrombogenic bonding nonetheless be desired to achieve desirable material surface antimicrobial/anti-fouling properties, the technology herein at least has the following advantages: (i) ionic bonding of antimicrobial/antithrombogenic agents onto cationic TPU polymer substrates to achieve non-leaching or controlled release of active agents; (ii) polymer substrates already have cationic functionalities for bonding of active agents and no priming (e.g., chemical or plasma treatments) of polymer substrates is needed, which would simplify medical device manufacturing process and significantly reduce conversion costs; iii) anionic antimicrobial and/or antithrombogenic agents would be ionically bonded not only on cationic TPU surface but also in the bulk cationic TPU for potential continuous and long-term antimicrobial/antithrombogenic agent supply to device surface.

The cationic modifier according to the claimed invention comprises one or more quaternary ammonium functional moieties. A non-limiting example of the cationic modifier with quaternary ammonium functional moiety includes bis(2-hydroxyethyl)dimethylammonium chloride (BHDAC). The cationic modifier may comprise more than one functional moieties.

In one or more embodiments, the cationic modifier is incorporated into the backbone. In an embodiment, the cationic modifier incorporated into the backbone comprises bis(2-hydroxyethyl)dimethylammonium chloride (BHDAC).

In one or more embodiments, the cationic modifier is incorporated both as a side chain and into the backbone, as discussed herein.

In one or more embodiments, the medical articles herein are effective to reduce thrombus formation and/or bacterial biofilm. In one or more embodiments, the medical articles passively reduce thrombus formation and/or bacterial biofilm formation due to inhibition of microbial growth by cationic quaternary ammonium and ionic repulsion of blood components.

The polyurethanes described herein may be fabricated into film, tubing, and other forms by conventional thermoplastic fabricating techniques including melt casting, compounding, extrusion/coextrusion, molding, etc. The polyurethane described herein may be used for PICCs, PIVCs, and CVCs. The polymer may have incorporated therein, as desired, conventional stabilizers, additives (e.g., a radiopaque filler), and/or processing aids. The amounts of these materials will vary depending upon the application of the polyurethane, but if present, are typically in amounts so in the range of from 0.1 to 50 weight percent of the final compound.

### POLYURETHANES INCLUDING LOW-SURFACE ENERGY MODIFYING OLIGOMERS

Optionally, the polyurethanes herein may further comprise low-surface energy modifying oligomers to provide further surface enhancements as described in commonly-assigned, co-pending U.S. Ser. Nos. 16/577824 and 16/577826, filed September 20, 2019 (WO 2020/068617 A1 and WO 2020/068619 A1). An advantage of low-surface energy modified polyurethane materials is that their non-sticking, hydrophobic surfaces can provide antimicrobial, self-lubricating and/or anti-fouling properties.

The polyurethanes including low-surface energy modifying oligomers may be produced by the reaction of: a diisocyanate, a diol chain extender, at least one polyglycol, an ionically-charged modifier, and a low-surface energy modifying oligomer. The modified polyurethanes comprise a hard segment content in the range of from 25% to 75% and a soft segment content in the range of from 75% to 25% by weight.

Polymerization of the polyurethane to include a low-surface energy modifying oligomer may be a one-step or a two-step copolymerization process. The process may require a catalyst, solvent, other additives, or a combination thereof. The synthesis can also be achieved by a variety of other synthesis techniques with or without catalyst/solvent understood by those skilled in the art.

The low-surface energy modifying oligomers contain functional moieties that migrate onto the polyurethane surface to render the resulting medical article desirable surface properties. Non-limiting examples of the low-surface energy modifying oligomer include fluoroether, silicone, or combination thereof. In one or more embodiments, the low-surface energy modifying oligomers have at least one, preferably two, alcohol moieties (C-OH).

A low-surface energy modifying oligomer for the backbone may comprise a diol-containing perfluoropolyether.

In one or more embodiments, the diol-containing perfluoropolyether has the following structure.

HO(CH₂CH₂O)ₚCH₂CF₂O(CF₂CF₂O)_{q}(CF₂O)ᵣCF₂CH₂(OCH₂CH₂)ₚOH

Wherein total of values for p+q+r are such that the fluorine content of the oligomer may be in the range of from 55% to 60% by weight and the average molecular weight of the oligomer is in the range of from 1500 to 2200 Da.

An exemplary diol-containing perfluoropolyether (PFPE) may be a commercial product sold under the trade name Fluorolink^{®} E10-H, which is a dialcohol-terminated, ethoxylated PFPE, with about 1,700 Da average molecular weight and about 57% w/w fluorine content.

A low-surface energy modifying oligomer as a side chain may comprise a monofunctional polysiloxane. In one or more embodiments, the monofunctional polysiloxane is a monodialcohol-terminated polydimethylsiloxane (PDMS) having the following structure. 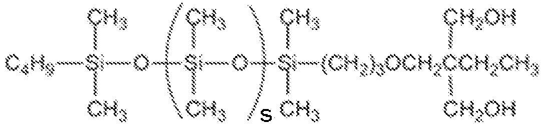 wherein, s may be in the range of from 5 to 200.

Exemplary monodialcohol-terminated polydimethylsiloxanes may be a commercial product sold under the product codes MCR-C61, MCR-C62 and MCR-C63. MCR-C62 has an average molecular weight of 5000 Da (s in range of 62-63), MCR-C61 has an average molecular weight of 1000 Da (s in range of 8-9), and MCR-C63 has an average molecular weight of 15,000 Da (s in range of 197-198). In one or more embodiments, the low-surface energy modifying oligomer for the as a side chain is MCR-C62.

### BONDING OF ACTIVE AGENTS WITH POLYURETHANE-BASED RESINS

In one or more embodiments, the polyurethane-based resin is bound to an anionic agent through ionic bonding. In various embodiments, the anionic agent comprises one or more of: an antimicrobial agent, a lubricating agent, and an antithrombotic agent.

Antimicrobial agents that can be used for bonding with cationic functional moieties of the polyurethane include any anionic antibiotics. Non-limiting anionic antibiotics include cloxacillin salt, cefoxitin salt, cefazolin salt, penicillin salt, or derivatives thereof. Non-limiting examples of the anionic antithrombogenic agents include heparin salt, or derivatives thereof. In addition, the skilled artisan will recognize that other anionic biocides and anticoagulants of either small molecules or macromolecules can also be used for bonding with cationic functional groups of the polyurethane.

Ionic bonding of active agents can be achieved by solution imbibing technique or bulk mixing (e.g., thermal compounding or solvent mixing) technique. As a result, anionic antimicrobial and/or antithrombogenic agents would be ionically bonded not only on cationic TPU surface but also in the bulk cationic TPU to render the resulting medical device desirable properties, including antimicrobial and anti-fouling.

In one or more embodiments, the medical articles herein are effective to provide antimicrobial and/or anti-fouling activity. In one or more embodiments, the medical articles actively provide enhanced surface properties including antimicrobial and/or anti-fouling activity.

### GENERAL PROCEDURE FOR POLYURETHANE SYNTHESIS

The polyurethanes discussed herein were prepared by a one-step copolymerization process using a pilot-scale polyurethane (PU) processor. No catalyst or solvent was used for this reaction. The polyglycol(s) (e.g., PTMEG), cationic modifier(s) (e.g., BHDAC, introduced as a cationic diol chain extender), and chain extender(s) (e.g., 1,4-butanediol) in the total amount of about 7.5 kg were charged into B tank (2.5 gallon full tank capacity with a recycle loop) of the PU processor with adequate mixing through a tank agitator at a set temperature until the solid cationic modifier was completed dissolved in the polyglycol/extender mixture; the diisocyanate (e.g., MDI, calculated amount to react out B tank diol mixture) was charged into A tank (2.5 gallon full tank capacity with a recycle loop) of the PU processor; during reaction, both B tank and A tank materials were pumped through their individual feeding lines at controlled feed rates to achieve an isocyanate index of 1.0 to 1.1; in one or more embodiments, the isocyanate index is 1.02; both the B and A streams were continuously injected through their respective injectors into a 8 cc mixing head with high rotor speed for adequate mixing and poured into silicone pans (covered with Teflon sheets); the entire PU processor system, including A/B tanks, fill/feed/recycle/drain lines, injectors and mixing head, was maintained at a temperature of 50 - 90 °C (various zone temperature controls) and the tanks were pulled under vacuum of < 100 mmHg during operation; the silicone pans filled with the PU reactants mixture passed through a 150 °F conveyor oven with 10 - 20 min of curing time to achieve complete reaction; the resulting white/yellow PU slab has a dimension of 7.7 in × 3.5 in × 0.3 in. The PU slabs were subsequently grinded into granulated forms for downstream compounding and extrusion/coextrusion processes.

The PU granulates/chips were extruded into ribbon sheets for material property characterizations.

The PU ribbon sheets can be extruded either from a single copolymer composition or from a blend of two or more different PU compositions. Blending/compounding approach can allow for quick creation and characterization of new PU compositions using the already existing PU copolymers. Even though the micro-domain structure and molecular weight distribution may be different using direct copolymerization approach compared to blending/compounding approach, it is expected that comparable material properties will result based on a comparable overall PU composition. In one or more embodiments, direct copolymerization approach was used for preparation of cationic PU ribbon compositions.

**Table I. Exemplary Formulations of Polyurethane Resins with the proviso that the ingredients total 100%.**

| **Table I** | I-A | **I-B** | I-C |
|---|---|---|---|
| Reactant | by weight | by weight | by weight |
| Diisocyanate | 24-75% | 24-70% | 24-65% |
| Total Polyglycol | 15-75% | 20-70% | 25-65% |
| Regular Diol Chain Extender | 0.01-25% | 0.01-25% | 0.01-25% |
| Cationic Modifier | 0.01-10% | 0.01-10% | 0.01-10% |
| Modifying Oligomer (Optional) | 0-10% | 0-10% | 0-10% |
| Hard Segment % | 25-75% | 30-70% | 35-65% |

### EXEMPLARY POLYURETHANE-BASED RESINS

Medical articles are formed from a polyurethane-based resin, which is a reaction product of the following ingredients: a diisocyanate; a diol chain extender; a polyglycol; and a cationic modifier comprising one or more quaternary ammonium functional group, wherein the cationic modifier is incorporated into a backbone, or both into a backbone and as a side chain. In one or more embodiments, the polyglycol is one or more polyalkylene glycols, which may comprise a polytetramethylene ether glycol. The resulting polyurethane-based resins are random copolymers based on the ingredients. A hard segment content is in the range of from 25% to 75% by weight, and a soft segment content of the resin is in the range of from 75% to 25% by weight.

Using the following ingredients, various polymer chain segments (A) - (C) are expected: the diisocyanate comprises 4,4'-diphenylmethane diisocyanate (MDI); the diol chain extender comprises 1,4-butanediol; the polyglycols comprise a polytetramethylene ether glycol (PTMEG) with average MW in the range of from 250 Da to 2900 Da (n = 3 - 40); and the cationic modifier comprises bis(2-hydroxyethyl)dimethylammonium chloride (BHDAC), which is introduced as a cationic diol chain extender and is part of the polyurethane hard segments. In one or more embodiments, the polyurethane-based resins are cationic polyurethane-based resins, which are random copolymers comprising the following chain segments of (A), (B) and (C).

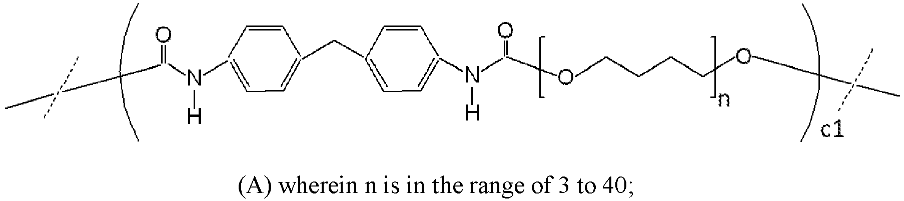

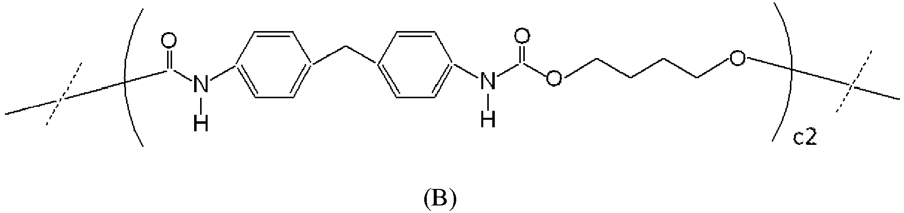

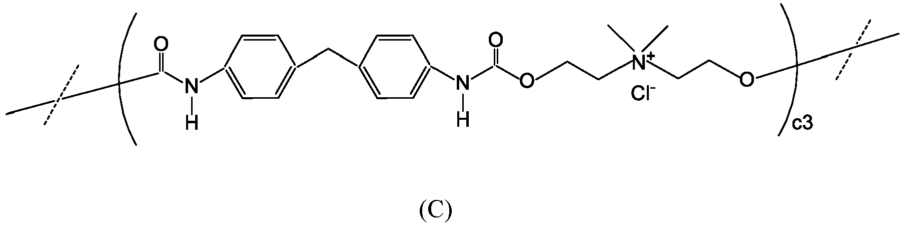

In one or more embodiments, the polyurethane-based resins are cationic polyurethane-based resins including a low-surface energy modifying oligomer, which are random copolymers comprising various polymer chain segments (A) - (E) using the following ingredients: the diisocyanate comprises 4,4'-diphenylmethane diisocyanate (MDI); the diol chain extender comprises 1,4-butanediol; the polyglycols comprise a polytetramethylene ether glycol (PTMEG) with average MW in the range of from 250 Da to 2900 Da (n = 3 - 40); the cationic modifier comprises bis(2-hydroxyethyl)dimethylammonium chloride (BHDAC); and the low-surface energy modifying oligomers comprise a diol-containing perfluoropolyether and/or a monofunctional polysiloxane. In one or more embodiments, the polyurethane-based resins are random copolymers comprising the following chain segments of (A), (B), (C); and one or both of (D) and (E). 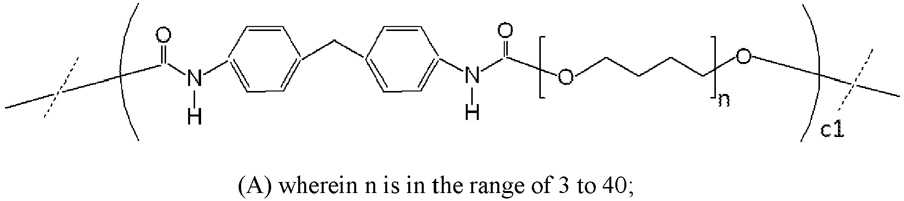 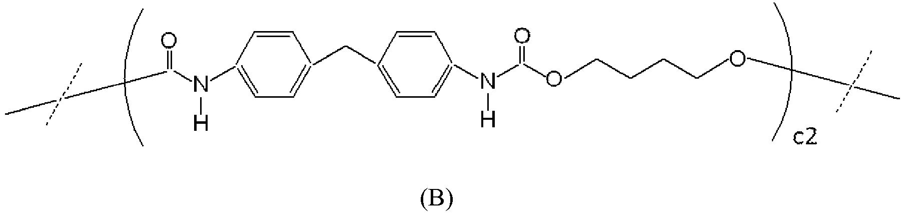 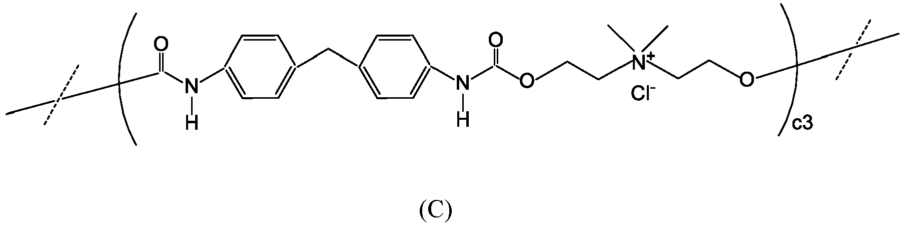 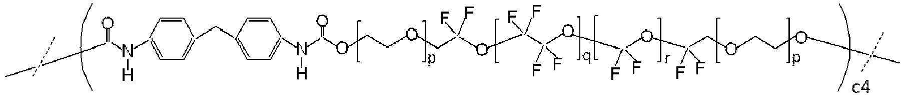 (D) wherein the total of p+q+r is such that the fluorine content of the oligomer is in the range of from 55% by weight to 60% by weight and the average molecular weight of the oligomer is in the range of from 1500 to 2200 Da;

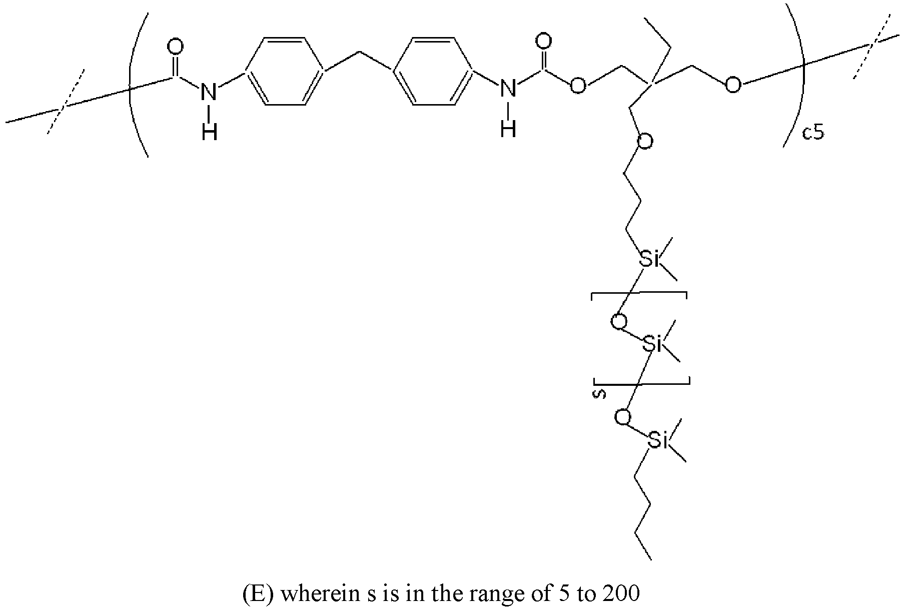

### MEDICAL ARTICLES OF POLYURETHANE

Medical articles may be any plastic part of a fluid path. Exemplary medical articles that may be formed by the polyurethanes disclosed herein may be a component of a catheter; a needle/needleless connector; or tubing. Exemplary devices are: central venous catheters, peripherally-inserted central catheters, and peripheral intravenous catheters. Catheter tubing can be formed through compounding and extrusion/coextrusion processes. During compounding, granulates of synthesized polyurethanes described herein, and an optional radiopaque filler are added into a twin-screw compounder simultaneously. The mix ratio can be controlled and adjusted by a gravimetric multiple-feeder system. The mixed polyurethane melt (conveying through multiple heating zones) continuously passes through a die, a quench tank, and is subsequently cut into regular-sized pellets by a puller-pelletizer. The collected pellets are used to be fed into an extruder/coextruder to form a catheter tube, depending on tubing's specific configuration.

Medical articles formed from cationic polyurethane resins disclosed herein can potentially possess inherent antimicrobial and/or anti-fouling surface properties, due to inhibition of microbial growth by cationic quaternary ammonium and ionic repulsion of blood components.

Antimicrobial agents that can be used for bonding with cationic functional moieties of the polyurethane include any anionic antibiotics, e.g., cloxacillin salt, cefoxitin salt, cefazolin salt, penicillin salt etc. Similarly, anionic antithrombogenic agents, e.g., heparin salt, can be ionically bonded with cationic functional moieties of the polyurethane to provide medical article desirable antithrombogenic properties. In addition, the skilled artisan will recognize that other anionic biocides and anticoagulants of either small molecules or macromolecules can also be used for bonding with cationic functional groups of the polyurethane. Ionic bonding of active agents can be achieved by solution imbibing technique or bulk mixing technique. In one or more embodiments, the bulk mixing technique comprises a thermal compounding technique and a solvent mixing technique. As a result, anionic antimicrobial and/or antithrombogenic agents would be ionically bonded not only on cationic TPU surface but also in the bulk cationic TPU to render the resulting medical device desirable properties, including antimicrobial and anti-fouling.

### EXAMPLES

### Example 1

Cationic thermoplastic polyurethane (TPU) resins were made in accordance with Table 2 by the one-step copolymerization process (no catalyst or solvent) using a pilot-scale polyurethane (PU) processor as described earlier in accordance with Exemplary Formulation I-C as shown above. Exemplary formulations had MDI as an aromatic diisocyanate, a combination of polytetramethylene ether glycols (PTMEGs with average molecular weight of 500 - 1000 Da), 1,4-butanediol as the chain extender, and bis(2-hydroxyethyl)dimethylammonium chloride (BHDAC) as the cationic modifier according to Table 2. No low-surface energy modifying oligomer was present. Reference polyurethane without a cationic modifier was made as well. Table 2 shows both the benchmark reference and the cationic TPU copolymer compositions.

**Table 2.**

| **Example** | **Total Hard Segment Content** | **Cationic Modifier** | **Location of Cationic Modifier** | **Cationic Modifier Content** |
|---|---|---|---|---|
| Q-PU-1 | 61.0 wt.% | BHDAC | Chain Extender Hard Segment | 0.34 wt.% |
| Q-PU-2 | 61.0 wt.% | BHDAC | Chain Extender Hard Segment | 0.96 wt.% |
| Q-PU-3 | 61.0 wt.% | BHDAC | Chain Extender Hard Segment | 2.51 wt.% |
| Reference PU-A | 61.0 wt.% | NONE | NONE | NONE |

Q-PU-2, Q-PU-3 and Reference PU-A were prepared by direct copolymerization in PU reactor, while Q-PU-1 was prepared by uniform blend of two different PUs (i.e., 35/65 wt.% blend of Q-PU-2 and Reference PU-A).

Table 3 shows gel temperatures and gel times for the copolymerization reactions according to Examples Q-PU-2, Q-PU-3, and Reference PU-A.

**Table 3.**

| **Example** | **Gel temperature (°C)** | **Gel time (second)** |
|---|---|---|
| Q-PU-2 | 179 | 54.9 |
| Q-PU-3 | 165 | 61.8 |
| Reference PU-A | 170 | 54.8 |

As Table 3 shows, incorporation of the cationic modifier BHDAC (introduced as a cationic diol chain extender) during copolymerization at 0.96 wt.% did not change the reaction rate and polymerization gel time significantly; however, introduction of 2.51 wt.% of cationic modifier BHDAC increased the polymerization gel time to 61.8 sec, indicating slower reaction.

### Example 2

### TESTING

**Calculation of Ion Exchange Capacity.** The ion exchange capacity (mmol/gm) of cationic TPUs can be easily calculated based on the copolymer compositions as shown in Table 4.

**Table 4.**

| **Example** | **Cationic Modifier Content in TPU Copolymer** | **Ion Exchange Capacity (mmol/gm)** |
|---|---|---|
| Q-PU-1 | 0.34 wt.% | 0.020 |
| Q-PU-2 | 0.96 wt.% | 0.057 |
| Q-PU-3 | 2.51 wt.% | 0.148 |
| Reference PU-A | NONE | 0 |

For examples of Table 2, TPU slabs (dimension of about 7.7 in × 3.5 in × 0.3 in) were produced from the above mentioned pilot-scale PU processor and conveyor oven curing system, which were subsequently ground into granulated forms and extruded into ribbon sheets for material physical property characterizations. The thickness of the ribbon sheets was 0.007 - 0.010 in.

**Tensile Property Testing.** Tensile properties of both the reference and the cationic PU ribbons (thickness of 0.007 - 0.010 in.) were characterized using Instron. The testing was performed at room conditions (23 °C, 50% RH, and > 40 h equilibration time), which is provided in Table 5 (mean of 10 measurements for each data).

**Table 5.**

| **EXAMPLE** | **Tensile at break (psi)-Elongation at break (%)** | **Tensile at 5% strain (psi)** | **Tensile at 25% strain (psi)** | **Tensile at 50% strain (psi)** | **Tensile at 100% strain (psi)** | **Tensile at 200% strain (psi)** | **Young's Modulus (MPa)** |
|---|---|---|---|---|---|---|---|
| Q-PU-1 | 11265.64-339.60 | 2114.62 | 2332.59 | 2661.73 | 3612.86 | 6139.32 | 482.76 |
| Q-PU-2 | 9892.94-356.36 | 1909.60 | 2157.55 | 2454.33 | 3261.04 | 5368.80 | 434.65 |
| Q-PU-3 | 7816.99 358.69 | 1284.46 | 1704.69 | 1974.41 | 2646.78 | 4357.26 | 278.19 |
| Reference PU-A | 11003.46 - 306.27 | 2317.78 | 2537.44 | 2904.74 | 3932.39 | 6707.76 | 528.77 |

Testing was also performed at body indwell conditions (37 °C, water equilibration for 4 hours), which is provided in Table 6 (mean of 10 measurements for each data). Soften ratio is defined according to the following Equation (1). $\begin{matrix}Soften Ratio = \\ \frac{Young 9 s Modulus at Room Conditions - Young 9 s Modulus at Body Indwell Conditions}{Young 9 s Modulus at Room Conditions} \times 100 %\end{matrix}$ Equation (1)

**Table 6.**

| **Example** | **Tensile at break (psi) - Elongation at break (%)** | **Tensile at 5% strain (psi)** | **Tensile at 25% strain (psi)** | **Tensile at 50% strain (psi)** | **Tensile at 100% strain (psi)** | **Tensile at 200% strain (psi)** | **Young's Modulus (MPa)** | **Soften Ratio (%)** |
|---|---|---|---|---|---|---|---|---|
| Q-PU-1 | 9873.69 - 397.00 | 418.37 | 951.35 | 1173.70 | 1606.89 | 3378.99 | 70.92 | 85.31 |
| Q-PU-2 | 8717.81 - 430.51 | 441.41 | 928.31 | 1099.16 | 1448.33 | 2812.57 | 75.78 | 82.57 |
| Q-PU-3 | 5348.29 - 399.22 | 345.10 | 724.89 | 843.84 | 1057.86 | 1857.94 | 59.44 | 78.63 |
| Reference PU-A | 9500.22 - 343.55 | 408.47 | 992.86 | 1268.98 | 1820.49 | 3970.41 | 62.66 | 88.15 |

Comparison of tensile properties of Reference PU-A with cationic TPUs Q-PU-2 and Q-PU-3 at room conditions shows that with introduction of cationic modifier BHDAC as part of the chain extender hard segment, both material ultimate tensile strength and material stiffness (Young's modulus) reduced, while material ultimate tensile strain did not change significantly.

Comparison of tensile properties of Reference PU-A with cationic TPUs Q-PU-2 and Q-PU-3 at body indwell conditions shows that with introduction of cationic modifier BHDAC as part of the chain extender hard segment, material ultimate tensile strength reduced, while material ultimate tensile strain and material stiffness (Young's modulus) did not change significantly, which resulted in reduced material soften ratio.

Overall, after introduction of cationic modifier BHDAC, the novel cationic TPUs still exhibited desirable tensile properties for medical device applications.

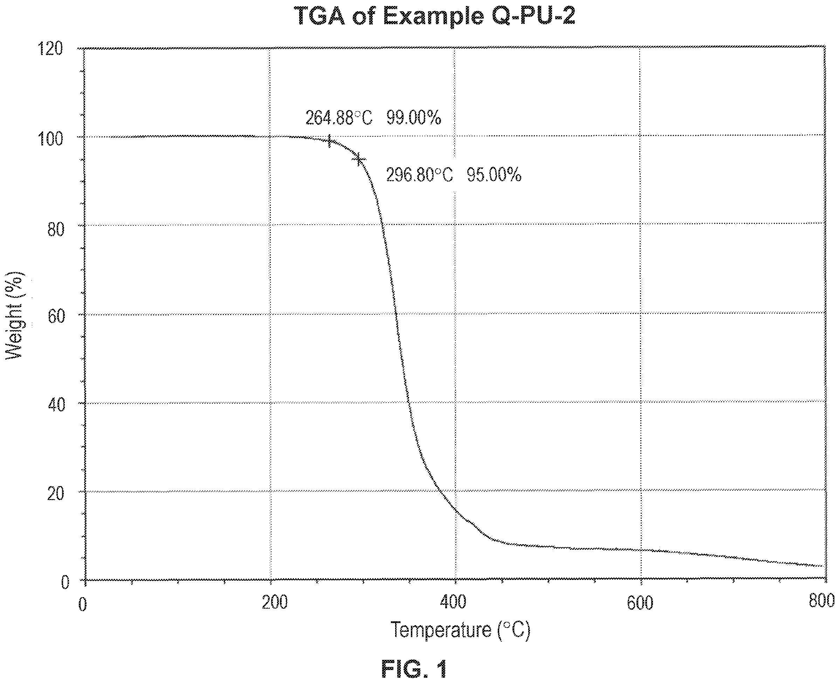

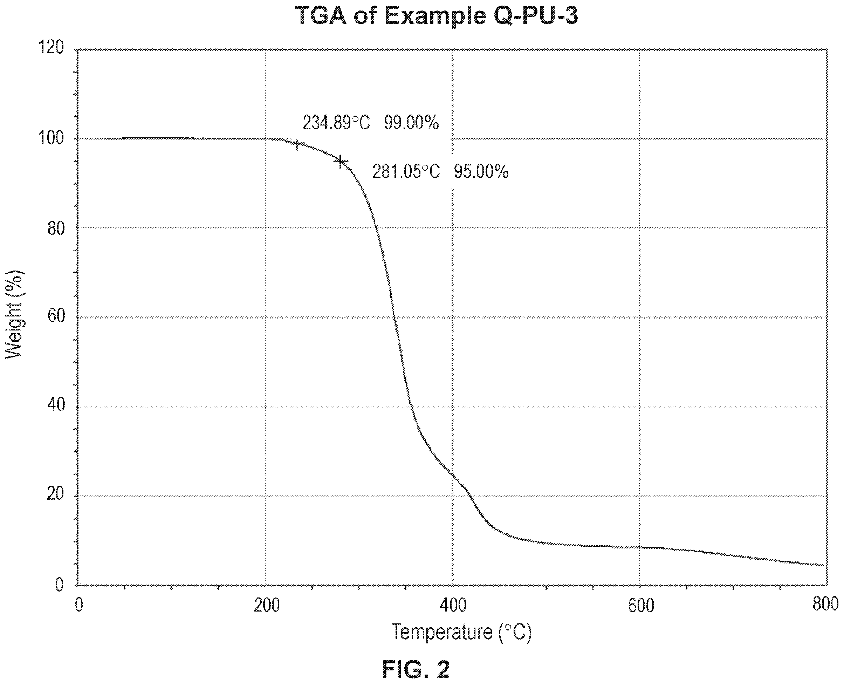

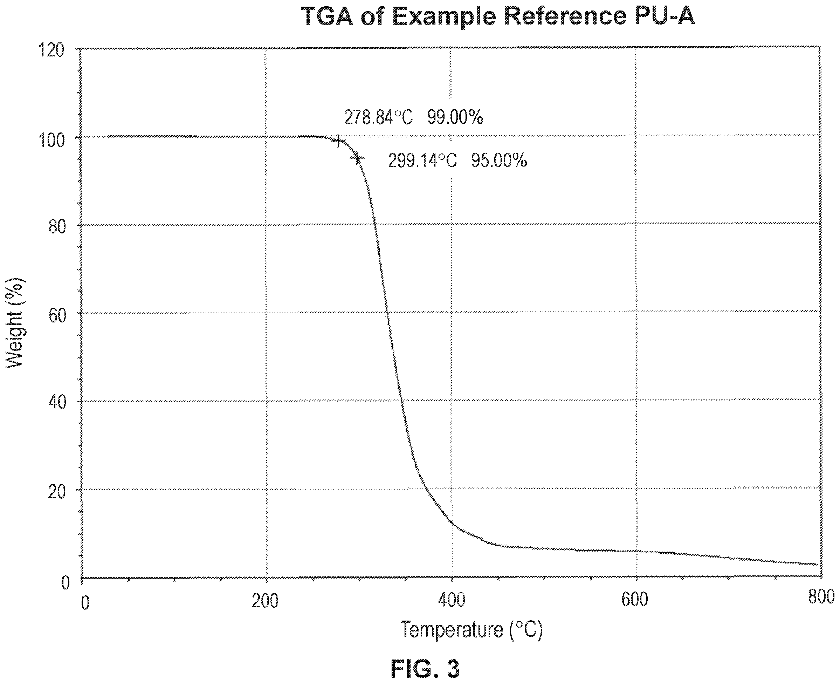

**Thermogravimetric Analysis (TGA).** The reference and inventive cationic TPU granulates/chips were analyzed using TA Instruments TGA Q500. For testing, 3 mg of each sample was heated from 25 °C to 800 °C at 10 °C/min in Nitrogen gas. FIGS. 1 & 2 show the TGA curves of the cationic TPUs Q-PU-2 and Q-PU-3, respectively. FIG. 3 shows the TGA curve of the Reference PU-A. Table 7 shows the degradation temperatures (based on 1% and 5% weight losses) of both the reference and inventive cationic TPU materials.

**Table 7.**

| **EXAMPLE** | **Degradation T at 1% of Weight Loss (°C)** | **Degradation T at 5% of Weight Loss (°C)** |
|---|---|---|
| Q-PU-2 | 264.88 | 296.80 |
| Q-PU-3 | 234.89 | 281.05 |
| Reference PU-A | 278.84 | 299.14 |

Table 7 shows that introduction and increase of cationic modifier BHDAC as part of the chain extender hard segment decreased material thermal degradation temperatures of the resulting cationic TPUs, presumably due to the thermal degradation of quaternary ammonium functional groups. These information are useful and can be referenced for compounding, ribbon and tubing extrusion of the new inventive cationic TPU materials as lower thermal processing temperatures may be required to prevent potential cationic TPU copolymer thermal degradation.

**Melt Flow Index.** The reference and inventive cationic TPU granulates/chips were characterized for melt flow indexes using a Zwick/Roell extrusion plastometer. The equipment has an extrusion barrel diameter of 9.55 mm (length of 170 mm) and a piston diameter of 9.48 mm (weight of 325 g). Five (5) g of each pre-dried (dried at 95 - 110 °C for over 12 hours) sample was used to perform the test at 210 °C with 5 kg of load weight and 300 seconds of preheat time. Table 8 shows the melt mass flow rate, melt volume flow rate and melt density of both the reference and inventive cationic TPU materials.

**Table 8.**

| **Example** | **Melt Mass Flow Rate (g/10 min)** | **Melt Volume Flow Rate (cm³/10 min)** | **Melt Density (g/cm³)** |
|---|---|---|---|
| Q-PU-2 | 20.56 | 19.65 | 1.046 |
| Q-PU-3 | 36.96 | 35.32 | 1.047 |
| Reference PU-A | 1.553 | 1.485 | 1.045 |

Table 8 shows that introduction and increase of cationic modifier BHDAC as part of the chain extender hard segment increased the melt flow of the resulting cationic TPUs significantly. These information are useful and can be referenced for compounding, ribbon and tubing extrusion of the new inventive cationic TPU materials as lower thermal processing temperatures may be required to achieve desirable melt flows.

**Molecular Weight.** The reference and inventive cationic TPU granulates/chips were characterized for molecular weight using Gel Permeation Chromatography / Multi Angle Light Scatter (GPC-MALS). Samples were dissolved in N,N-dimethylformamide, centrifuged, and diluted to 5 mg/mL. They were injected (200 microliters volume) into a mobile phase of N,N-dimethylformamide with 0.1 M LiBr and run through two (2) 300 mm Agilent 5 µm PLgel Mixed-C columns to separate them by molecular weight. Wyatt T-REX and Helios II detectors were used to measure light scattering and differential refractive index, respectively. Wyatt Astra was used to analyze the detector outputs and calculate molecular weight results. Polystyrene standards were used for calibration. Table 9 shows number average molecular weight (Mₙ), weight average molecular weight (M_{w}), and polydispersity index (PDI) of both the reference and inventive cationic TPU materials.

**Table 9.**

| **Example** | **Number Average Molecular Weight (Mn, Da)** | **Weight Average Molecular Weight (M_{w}, Da)** | **Polydispersity Index (PDI, M_{w}/Mₙ)** |
|---|---|---|---|
| Q-PU-2 | 21784 | 41389 | 1.900 |
| Q-PU-3 | 11355 | 35753 | 3.149 |
| Reference PU-A | 32103 | 54358 | 1.693 |

Table 9 shows that with introduction of cationic modifier BHDAC as part of the chain extender hard segment, the resulting cationic TPU copolymer molecular weight reduced compared to Reference PU-A, but still pretty high (Mₙ > 10K Da) to provide material desirable tensile properties (as data shown in previous tensile property session); in addition, higher PDI was observed for these cationic TPUs.

Although the invention herein has been described with reference to particular embodiments, it is to be understood that these embodiments are merely illustrative of the principles and applications of the present invention.

## Claims

1. A medical article formed from a polyurethane-based resin, which is a reaction product of the following ingredients:
a diisocyanate;
a diol chain extender;
a polyglycol; and
a cationic modifier comprising one or more quaternary ammonium functional groups, wherein the cationic modifier is (i) incorporated into a backbone or (ii) both incorporated into a backbone and as a side chain of the polyurethane-based resin formed by the diisocyanate, the polyglycol, and the diol chain extender;
wherein the polyurethane-based resin is a random copolymer based on the ingredients and wherein the polyurethane-based resin has a hard segment content in a range of from 25% to 75% by weight and a soft segment content of the resin is in a range of from 75% to 25% by weight.

2. The medical article of claim 1, wherein the cationic modifier comprises: bis(2-hydroxyethyl)dimethylammonium chloride (BHDAC).

3. The medical article of claim 1, wherein the cationic modifier is present in an amount of greater than or equal to 0.01 weight percent of the overall composition of the polyurethane-based resin or wherein the cationic modifier is present in an amount of less than or equal to 10 weight percent of the overall composition of the polyurethane-based resin.

4. The medical article of claim 1, wherein the diisocyanate is selected from the group consisting of: an aliphatic diisocyanate, alicyclic diisocyanate and an aromatic diisocyanate, preferably wherein the diisocyanate is selected from the group consisting of: 4,4'-diphenylmethane diisocyanate (MDI), toluene diisocyanate (TDI), isophorone diisocyanate (IPDI), methylene-bis(4-cyclohexylisocyanate) (HMDI), and combinations thereof.

5. The medical article of claim 1, wherein the diol chain extender is selected from the group consisting of: ethylene glycol, 1,3-propylene glycol, 1,4-butanediol, neopentyl glycol, and alicyclic glycols having up to 10 carbon atoms.

6. The medical article of claim 1, wherein the polyglycol is selected from the group consisting of: polyalkylene glycol, polyester glycol, polycarbonate glycol, and combinations thereof.

7. The medical article of claim 1, wherein the polyglycol comprises the polyalkylene glycol, preferably wherein the polyalkylene glycol comprises a polytetramethylene ether glycol.

8. The medical article of claim 1, wherein the ingredients of the reaction product consist essentially of:
4,4'-diphenylmethane diisocyanate (MDI) as the diisocyanate;
1,4-butanediol as the diol chain extender;
a polytetramethylene ether glycol as the polyglycol; and
bis(2-hydroxyethyl)dimethylammonium chloride (BHDAC) as the cationic modifier.

9. The medical article of claim 1, wherein the polyurethane-based resin is bound to an anionic agent through ionic bonding.

10. The medical article of claim 9, wherein the ionic bonding is achieved by a technique comprising a solution imbibing technique or a bulk mixing technique, preferably wherein the bulk mixing technique comprises a thermal compounding technique and a solvent mixing technique.

11. The medical article of claim 9, wherein the anionic agent comprises one or more of: an antimicrobial agent, a lubricating agent, and an antithrombotic agent, preferably wherein the anionic agent comprises one or more of: cloxacillin salt, cefoxitin salt, cefazolin salt, penicillin salt, and heparin salt.

12. The medical article of claim 1, wherein the ingredients of the reaction product further comprise: a low-surface energy modifying oligomer incorporated into a backbone, as a side chain, or both of the polyurethane-based resin formed by the diisocyanate, the polyglycol, the cationic modifier, and the diol chain extender, wherein the modifying oligomer has an alcohol (C-OH) moiety and a functional moiety, and wherein the functional moiety comprises a fluoroether, a silicone, or a combination thereof.

13. The medical article of claim 12, wherein the low-surface energy modifying oligomer is present in an amount ranging from about 0.1 to about 10 weight percent of the overall composition of the polyurethane-based resin.

14. The medical article of claim 1, wherein the polyurethane-based resin is a random copolymer comprising chain segments of (A), (B), and (C) as follows: 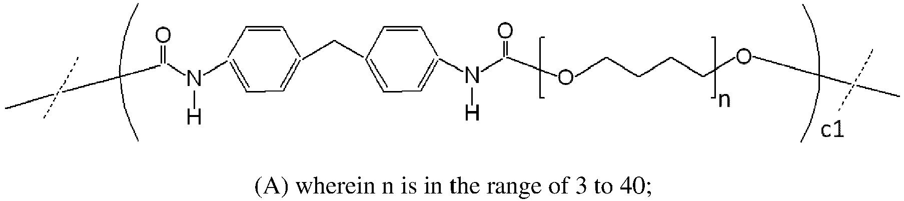 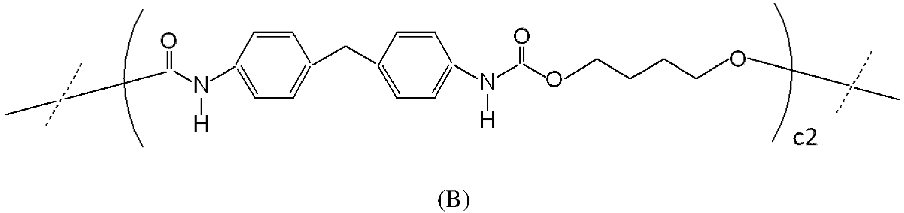 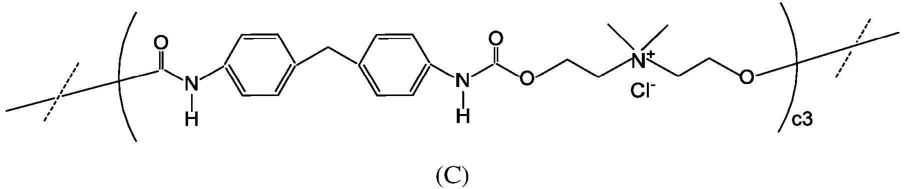
wherein chain segment (C) is (i) incorporated into a backbone or (ii) both incorporated into a backbone and as a side chain of the polyurethane-based resin, and
wherein the polyurethane-based resin has an overall ion exchange capacity of 0.01 to 1 mmol/g.

## Patentansprüche

1. Medizinischer Artikel, gebildet aus einem Harz auf Polyurethanbasis, das ein Reaktionsprodukt der folgenden Bestandteile ist:
ein Diisocyanat;
ein Diol-Kettenverlängerer;
ein Polyglycol; und
ein kationischer Modifikator, der eine oder mehrere funktionelle quartäre Ammoniumgruppen umfasst, wobei der kationische Modifikator (i) in ein Gerüst oder (ii) sowohl in ein Gerüst als auch in eine Seitenkette des Harzes auf Polyurethanbasis, welches durch das Diisocyanat, das Polyglycol und den Diol-Kettenverlängerer gebildet ist, eingebaut ist;
wobei das Harz auf Polyurethanbasis ein statistisches Copolymer auf der Basis der Bestandteile ist und wobei das Harz auf Polyurethanbasis einen Gehalt an hartem Segment im Bereich von 25 bis 75 Gew.-% aufweist und der Gehalt an weichem Segment des Harzes im Bereich von 75 bis 25 Gew.-% liegt.

2. Medizinischer Artikel gemäß Anspruch 1, wobei der kationische Modifikator Bis(2-hydroxyethyl)dimethylammoniumchlorid (BHDAC) umfasst.

3. Medizinischer Artikel gemäß Anspruch 1, wobei der kationische Modifikator in einer Menge von größer oder gleich 0,01 Gewichtsprozent der Gesamtzusammensetzung des Harzes auf Polyurethanbasis vorhanden ist oder wobei der kationische Modifikator in einer Menge von kleiner oder gleich 10 Gewichtsprozent der Gesamtzusammensetzung des Harzes auf Polyurethanbasis vorhanden ist.

4. Medizinischer Artikel gemäß Anspruch 1, wobei das Diisocyanat aus der Gruppe ausgewählt ist, die aus einem aliphatischen Diisocyanat, einem alicyclischen Diisocyanat und einem aromatischen Diisocyanat besteht, wobei vorzugsweise das Diisocyanat aus der Gruppe ausgewählt ist, die aus 4,4'-Diphenylmethandiisocyanat (MDI), Toluoldiisocyanat (TDI), Isophorondiisocyanat (IPDI), Methylenbis(4-cyclohexylisocyanat) (HMDI) und Kombinationen davon besteht.

5. Medizinischer Artikel gemäß Anspruch 1, wobei der Diol-Kettenverlängerer aus der Gruppe ausgewählt ist, die aus Ethylenglycol, 1,3-Propylenglycol, 1,4-Butandiol, Neopentylglycol und alicyclischen Glycolen mit bis zu 10 Kohlenstoffatomen besteht.

6. Medizinischer Artikel gemäß Anspruch 1, wobei das Polyglycol aus der Gruppe ausgewählt ist, die aus Polyalkylenglycol, Polyesterglycol, Polycarbonatglycol und Kombinationen davon besteht.

7. Medizinischer Artikel gemäß Anspruch 1, wobei das Polyglycol ein Polyalkylenglycol umfasst, wobei vorzugsweise das Polyalkylenglycol ein Polytetramethylenetherglycol umfasst.

8. Medizinischer Artikel gemäß Anspruch 1, wobei die Bestandteile des Reaktionsprodukts im Wesentlichen aus Folgenden bestehen:
4,4'-Diphenylmethandiisocyanat (MDI) als Diisocyanat;
1,4-Butandiol als Diol-Kettenverlängerer;
ein Polytetramethylenetherglycol als Polyglycol; und
Bis(2-hydroxyethyl)dimethylammoniumchlorid (BHDAC) als kationischem Modifikator.

9. Medizinischer Artikel gemäß Anspruch 1, wobei das Harz auf Polyurethanbasis durch ionische Bindung an ein anionisches Agens gebunden ist.

10. Medizinischer Artikel gemäß Anspruch 9, wobei die ionische Bindung durch eine Methode erreicht wird, die eine Lösungstränkmethode oder eine Massenmischmethode umfasst, wobei vorzugsweise die Massenmischmethode eine thermische Compoundiermethode und eine Lösungsmittelmischmethode umfasst.

11. Medizinischer Artikel gemäß Anspruch 9, wobei das anionische Agens eines oder mehrere aus einem antimikrobiellen Mittel, einem Gleitmittel und einem Antithrombosemittel umfasst, wobei vorzugsweise das anionische Agens eines oder mehrere aus einem Cloxacillin-Salz, einem Cefoxitin-Salz, einem Cefazolin-Salz, einem Penicillin-Salz und einem Heparin-Salz umfasst.

12. Medizinischer Artikel gemäß Anspruch 1, wobei die Bestandteile des Reaktionsprodukts ferner ein modifizierendes Oligomer mit niedriger Oberflächenenergie umfassen, das in ein Gerüst, eine Seitenkette oder beides des Harzes auf Polyurethanbasis, welches durch das Diisocyanat, das Polyglycol, den kationischen Modifikator und den Diol-Kettenverlängerer gebildet ist, eingebaut ist, wobei das modifizierende Oligomer eine Alkohol(C-OH)-Struktureinheit und eine funktionelle Struktureinheit aufweist und wobei die funktionelle Struktureinheit einen Fluorether, ein Silikon oder eine Kombination davon umfasst.

13. Medizinischer Artikel gemäß Anspruch 12, wobei das modifizierende Oligomer mit niedriger Oberflächenenergie in einer Menge im Bereich von etwa 0,1 bis etwa 10 Gewichtsprozent der Gesamtzusammensetzung des Harzes auf Polyurethanbasis vorhanden ist.

14. Medizinischer Artikel gemäß Anspruch 1, wobei das Harz auf Polyurethanbasis ein statistisches Copolymer ist, das Kettensegmente (A), (B) und (C) wie folgt umfasst: 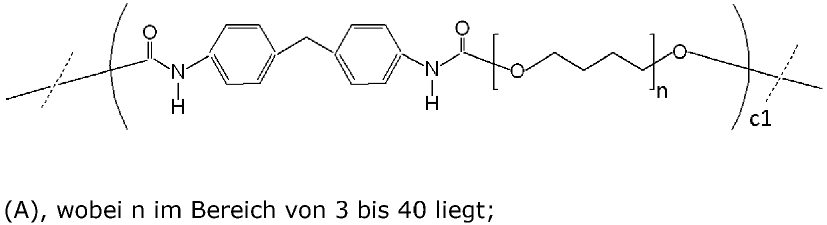 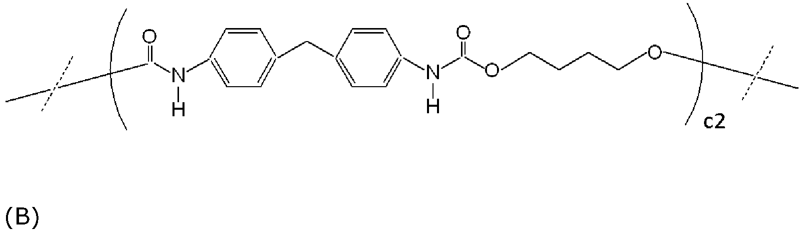 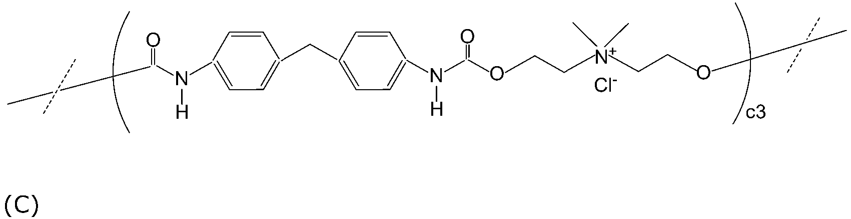
wobei das Kettensegment (C) (i) in ein Gerüst oder (ii) sowohl in ein Gerüst als auch in eine Seitenkette des Harzes auf Polyurethanbasis eingebaut ist und
wobei das Harz auf Polyurethanbasis eine Gesamtionenaustauschkapazität von 0,01 bis 1 mmol/g aufweist.

## Revendications

1. Article médical formé à partir d'une résine à base de polyuréthane, qui est un produit de réaction des ingrédients suivants :
un diisocyanate ;
un allongeur de chaîne diol ;
un polyglycol ; et
un modificateur cationique comprenant un ou plusieurs groupes fonctionnels ammonium quaternaire,
dans lequel le modificateur cationique est (i) incorporé dans un squelette ou (ii) à la fois incorporé dans un squelette et en tant que chaîne latérale de la résine à base de polyuréthane formée par le diisocyanate, le polyglycol, et l'allongeur de chaîne diol ;
dans lequel la résine à base de polyuréthane est un copolymère statistique à base des ingrédients, et dans lequel la résine à base de polyuréthane a une teneur en segments durs se trouvant dans la plage allant de 25% à 75% en poids et une teneur en segments souples de la résine se trouve dans la plage allant de 75% à 25% en poids.

2. Article médical selon la revendication 1, dans lequel le modificateur cationique comprend : du chlorure de bis(2-hydroxyéthyl)diméthylammonium (BHDAC).

3. Article médical selon la revendication 1, dans lequel le modificateur cationique est présent en une quantité supérieure ou égale à 0,01 pour cent en poids de la composition totale de la résine à base de polyuréthane, ou dans lequel le modificateur cationique est présent en une quantité inférieure ou égale à 10 pour cent en poids de la composition totale de la résine à base de polyuréthane.

4. Article médical selon la revendication 1, dans lequel le diisocyanate est choisi dans le groupe constitué par : un diisocyanate aliphatique, un diisocyanate alicyclique et un diisocyanate aromatique, de préférence dans lequel le diisocyanate est choisi dans le groupe constitué par : le diisocyanate de 4,4'-diphénylméthane (MDI), le diisocyanate de toluène (TDI), le diisocyanate d'isophorone (IPDI), le méthylène-bis(4-cyclohexylisocyanate) (HMDI), et leurs combinaisons.

5. Article médical selon la revendication 1, dans lequel l'allongeur de chaîne diol est choisi dans le groupe constitué par : l'éthylène glycol, le 1,3-propylène glycol, le 1,4-butanediol, le néopentyl glycol et des glycols alicycliques ayant jusqu'à 10 atomes de carbone.

6. Article médical selon la revendication 1, dans lequel le polyglycol est choisi dans le groupe constitué par : le polyalkylène glycol, le polyester glycol, le polycarbonate glycol et leurs combinaisons.

7. Article médical selon la revendication 1, dans lequel le polyglycol comprend le polyalkylène glycol, de préférence dans lequel le polyalkylène glycol comprend un polytétraméthylène éther glycol.

8. Article médical selon la revendication 1, dans lequel les ingrédients du produit de réaction consistent essentiellement en :
du diisocyanate de 4,4'-diphénylméthane (MDI) en tant que diisocyanate ;
du 1,4-butanediol en tant qu'allongeur de chaîne diol ;
un polytétraméthylène éther glycol en tant que polyglycol ; et
du chlorure de bis(2-hydroxyéthyl)diméthylammonium (BHDAC) en tant que modificateur cationique.

9. Article médical selon la revendication 1, dans lequel la résine à base de polyuréthane est liée à un agent anionique par une liaison ionique.

10. Article médical selon la revendication 9, dans lequel la liaison ionique est obtenue par une technique comprenant une technique d'imprégnation en solution ou une technique de mélange en masse, de préférence dans lequel la technique de mélange en masse comprend une technique de mélangeage à chaud (compoundage thermique) et une technique de mélange dans un solvant.

11. Article médical selon la revendication 9, dans lequel l'agent anionique comprend un ou plusieurs parmi : un agent antimicrobien, un agent lubrifiant et un agent antithrombotique, de préférence dans lequel l'agent anionique comprend un ou plusieurs parmi : un sel de cloxacilline, un sel de céfoxitine, un sel de céfazoline, un sel de pénicilline et un sel d'héparine.

12. Article médical selon la revendication 1, dans lequel les ingrédients du produit de réaction comprennent en outre : un oligomère modificateur à faible énergie de surface incorporé dans un squelette, en tant que chaîne latérale, ou les deux, de la résine à base de polyuréthane formée par le diisocyanate, le polyglycol, le modificateur cationique et l'allongeur de chaîne diol, dans lequel l'oligomère modificateur a un groupement alcool (C-OH) et un groupement fonctionnel, et dans lequel le groupement fonctionnel comprend un fluoroéther, un silicone ou une combinaison de ceux-ci.

13. Article médical selon la revendication 12, dans lequel l'oligomère modificateur à faible énergie de surface est présent en une quantité allant d'environ 0,1 à environ 10 pour cent en poids de la composition totale de la résine à base de polyuréthane.

14. Article médical selon la revendication 1, dans lequel la résine à base de polyuréthane est un copolymère statistique comprenant des segments de chaîne (A), (B) et (C) comme suit : 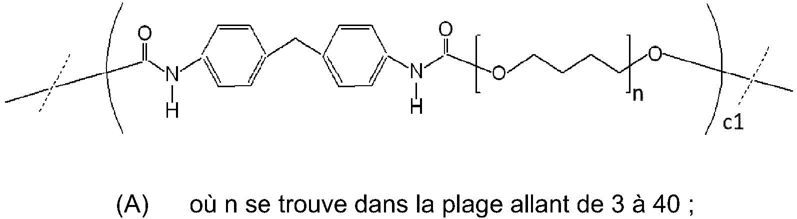 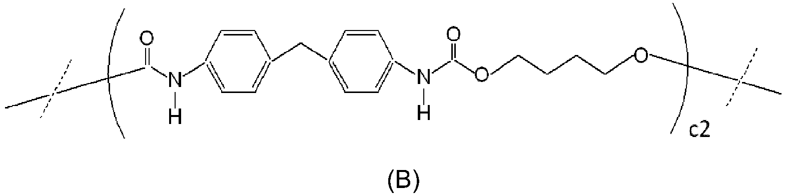 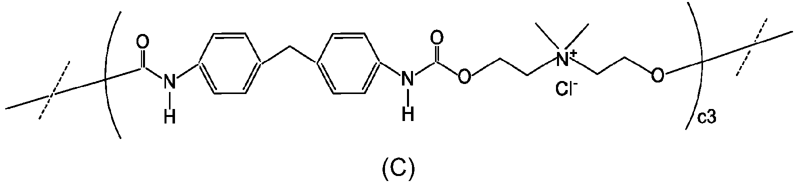
dans lequel le segment de chaîne (C) est (i) incorporé dans un squelette ou (ii) à la fois incorporé dans un squelette et est présent en tant que chaîne latérale de la résine à base de polyuréthane, et
dans lequel la résine à base de polyuréthane présente une capacité d'échange d'ions globale allant de 0,01 à 1 mmol/g.
